(19) Europäisches Patentamt
European Patent Office
Office européen des brevets

(11) Publication number : **0 456 373 A1**

(12) **EUROPEAN PATENT APPLICATION**

(21) Application number : **91303648.9**

(22) Date of filing : **23.04.91**

(51) Int. Cl.[5] : **C07C 6/04**, B01J 31/12

(30) Priority : **03.05.90 FI 902228**

(43) Date of publication of application :
**13.11.91 Bulletin 91/46**

(84) Designated Contracting States :
**AT BE CH DE DK ES FR GB GR IT LI LU NL SE**

(71) Applicant : **NESTE OY**
**Keilaniemi**
**SF-02150 Espoo (FI)**

(72) Inventor : **Hietala, Jukka**
**Luhtitie 2 B 5**
**SF-06400 Porvoo (FI)**
Inventor : **Knuuttila, Pekka**
**Torpparintie 4**
**SF-06400 Porvoo (FI)**

(74) Representative : **Lamb, John Baxter et al**
**MARKS & CLERK 57-60 Lincoln's Inn Fields**
**London WC2A 3LS (GB)**

(54) **A catalyst for a metathesis reaction of olefines and a method for preparing the same.**

(57) A catalyst system for metathesis reactions of olefines, especially of propylene, which system comrpises a metal carbene complex on a solid carrier, in which the metal carbene complex has the formula :

$$M(O)_y(RCH_2O)_mX_{n9}$$

wherein M is W or Mo ; R is t-butyl ; y is 0 or 1 ; and
when y is o, m = 3 or 4, X is Br and n is from 5 to m ; and
when y is 1, m is from 1 to 4, X = Cl and n is from 4 to m.

EP 0 456 373 A1

The invention relates to a catalyst for a metathesis reaction of olefines and a method for preparing the same.

In the olefine chemistry, the metathesis describes the mutual exchange of carbon atoms between a double-bond pair. These reactions include e.g. the metathesis of olefines (OM), which can generally be described e.g. by means of the following reaction equation:

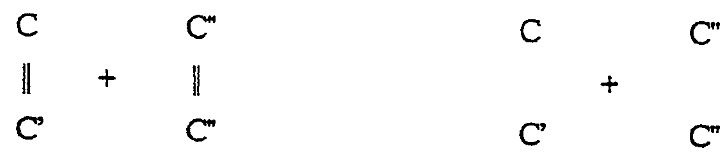

The metathesis reactions of olefines are not spontaneous, but they require a catalyst system containing a transitionmetal compound, which is preferably in contact with another compound (a cocatalyst) and sometimes with a third one (a promoter). The reactions are normally balance reactions, which can reach a balanced state in a couple of seconds with a good catalyst. Wolfram (W), molybdenum (Mo) and rhenium (Re) based catalysts are especially efficient, and essential are also several Ti, Nb, Ta, Re, Ru, Os and Ir systems. It is believed that the reaction is coordinatively activated by an unsaturated metal-carbene complex. The result of the reaction is also affected by the choice of a solvent, when there is a need to avoid side reactions, such as isomerization, alcylation, cyclization and addition over the double bond. For example halogenized solvents, such as chlorobenzene, are preferable solvents. It is known that the catalyst complex is carbene in solution systems and most likely also in reactios occurring with a solid carrier. Almost unexceptionally, this complex is not formed before the reaction conditions, wherein a small part of the metal changes into an active form.

Also, some metal carbenes operating without activation have been patented as a metathesis catalyst acting in a homogenous liquid phase. In general, in a heterogenous system is used a metal oxide e.g. with a silica or alumina carrier as well possibly a cocatalyst and a promoter. Typical systems include e.g. $Wo_3/SiO_2$, $MoO_3/Al_2O_3$ and $Re_2/Al_2O_3$.

In the US patent no. 4 686 314, a catalyst on an alkoxide modified carrier is described as being used in a metathesis reaction of olefines, which catalyst includes rhenium, molebdenum, wolfram and possibly a promoter, e.g. $Al(OCH(CH_2CH_3)(CH_3))_3$.

In the US patent no. 3 855 340, a catalyst has been described for a dismutation reaction of olefines according to the formula $X/O/_n/O\text{-}R/_{1-2n}$, wherein X is wolfram or molybdenum, and R is an alkyl group or a hydrocarbon residue. The catalyst acts in the presence of a halogenized organoaluminum compound (reduction).

In the DE publication print no. 2 213 948 is described for a conversion of olefines a catalyst according to the formula $R_mMX_p$ in an inert matrix material, wherein M is molybdenum, wolfram or rhenium, R is hydrocarbon and X is a univalent ligand, e.g. a halogen.

The inventors have invented new catalyst complexes, which have not previously been used for catalyzing metathesis reactions.

The catalyst system in accordance with the invention is mainly characterized in that said metal carbene complex has the formula:

$$M(O)_y(RCH_2O)_mX_n,$$

wherein M is W or Mo, R is t-butyl, y is 0 or 1, and
when y is o, m = 3-4, X is Br and n is 5-m, and
when y is 1, m is 1-4, X = Cl and n is 4-m.

It has been observed that the pure or modified silica of the complexes according to the invention has active metathesis catalysts.

Advantages of the catalysts according to the invention with respect to known metathesis catalysts include:

– Owing to organic ligands, the complexes dissolve well in non-polar solvents in contrast to wolfram salts used conventionally. This results in an easy impregnation, and the distribution of the metal on the carrier is uniform.

– Part of the complexes are sublimating complexes, and they can thus be brought to the carrier also by evaporazation.

– The catalyst is activated by nitrogen without an auxiliary catalyst (no air-sensitive and often pyrophoric organometal compounds, which are difficult to handle, are needed). The catalyst is activated in the reaction conditions (propylene, 400°C) without any pretreatment (the process is simpled, and no separate activation gases or regeneration gases are needed).

The preferred embodiments of the invention have the characteristics of the subclaims.

The invention is next illustrated in detail by describing the synthesis of the catalyst and the performance of the metathesis reaction itself.

**The synthesization of a metal complex according to invention and its impregnation from an organic solvent**

Synthesis

Metal complexes were synthesized from wolfram(IV)oxychloride $WOCl_4$ by substituting chloroatoms, by neopentoxide groups and oxygen by bromine or chlorine. The neopentoxide groups were derived from neopentyl alcohol ($(CH_3)_3CH_2OH$) and bromine from aluminium bromide ($ALBr_3$).

The structures of the complexes and the details of the synthesis have been described in the examples below. The syntheses were performed in a nitrogen atmosphere by using dried newly-distilled solvents.

The syntheses were performed in Schlenk vessels, which were in contact via a two-way cock with a separate vacuum line and a nitrogen line. An about 1 mbar vacuum was achieved in the vacuum line by means of an oil pump (Edwards 8). The line contains before the oil pump a cold trap, which is cooled by liquid nitrogen and in which the solvent fumes freeze. Pure bottle nitrogen was led to the nitrogen line, which nitrogen was as a precaution also dried with concentrated sulphur acid and neutralized with potassium hydroxide.

In all work phases were used vessels provided with cocks, which had been evacuated by opening a way to the vacuum line and then again filled with nitrogen by opening a way to the nitrogen line. When the vessels were open, a nitrogen atmosphere was maintained by means of a flow-through.

A separated synthesis product was dissolved in a small amount on pentane and transferred to a flask, in which a carrier had been weighed. The amount of the solvent used only slightly exceeded the amount the carrier was able absorb. The solvent was evaporated and it was again added several times for ensuring a uniform distribution of the dissolved complex. Finally, the catalyst was dried in a vacuum. It was loaded into a reactor tube in a nitrogen bag.

As solvents were used dietyl ether, pentane and dichloromethane, which were kept under nitrogen in a flask containing a drier. The necessary quantity of the solvent was distilled from the flask directly into the reaction vessel. Ether was dried with a potassium benzophenone mixture and dicloromethane and pentane with phosphor pentoxide.

NMR-spectra ($^{13}C$, $^{1}H$) and mass spectra were run from the metal complexes and metal assays of the catalysts were performed with an atom absorption spectrometer.

**Example 1**

1.03 g (3.0 mmol) of wolfram(IV)oxychloride were weighed in a nitrogen bag and dissolved in 100 ml of dietyl ether. When nearly all was dissolved, 1.20 g (15 mmol) of neopentyl alcohol dissolved in 10 ml of dietyl ether were added in small quantities. It was immediately observed that the orange color of the solution had disappeared. The solution was allowed to react during a moderate mixing for one hour.

The residue was evaporated and extracted several times with a pentane-dietyl ether mixture (ca. 1:1). 1,51 g of bluish oil was obtained as an evaporation residue of the extraction solution. The product was a mixture of compounds $W(O)(t\text{-}BuCH_2O)_mCl_n$, wherein m = 1 - 4, n = 4 - m (MS).

**Example 2**

1.50 g (4.4 mmol) of wolfram(IV)oxychloride were weighed in a nitrogen bag and dissolved in 120 ml of dietyl ether. When nearly all was dissolved, 1.60 g (18 mmol) of neopentyl alcohol was added in small quantities. The solution was mixed for about one hour.

The evaporation residue was dissolved, whereby a complex according to Example 1 was obtained. It was dissolved in 100 ml of dichloromethane and injected in 4.4 ml (4.4 mmol) of a dibromium methane solution of $AlBr_3$ by simultaneously mixing the solution. The mixing continued for ca. 20 hours (a long reaction time is not necessary).

The solution was evaporated and extracted several times with pentane. 2.45 g of an orange solid substance was obtained as an extraction residue. The product is in a solid state most likely a dimer complex ($W(O)(t\text{-}BuCH_2O)_3Br_2$ (MS, 1H-NMR, 13C-NMR). The yield was ca. 90%.

The product could be sublimated (60-70°C, 1 mmHg) without a change in its structure.

Reference catalysts were also prepared for showing the functionability of the catalyst in comparison with known products.

Reference catalysts ($WO_3(SiO_2)$:

**Examples 3 and 4**

4.5 g of ammonium wolframate $(NH_4)_2WO_4$ was weighed and distilled water was added by simultaneously mixing and sometimes warming up. The salt was dissolved, when ca. 200 ml of water had been added (a saturated solution in room temperature).

Said solution was mixed with 45.58 g of silica (PQ CS-1231, 0.6-1.6 mm) at 60-80°C for one hour. Most of the water was evaporated during three hours in a vacuum by sometimes warming up (70°C), the remainder in a heating chamber in an evaporation bowl (105°C). The catalyst was non-homogenous (it contained loose residue and the wolfram content varied between 4.5-6%).

In Example 4, an activator (1.5% MgO) was added to the catalyst.

Testing of catalysts

The catalysts were tested in a microreactor by leading purified propylene through a catalyst bed containing 0.2-0.5 g of catalyst. The propylene had been purified by removing the moisture by means of molecular sieves and oxygen by means of BASF's copper catalyst. Chrompack's capillary column (PLOT Fused Silica, liquid phase $Al_2O_3$/KCl, 50 x 0.32 mm) was used in the gas chromatography. During the runs, the propylene flow and the temperature of the reactor tube was often changed for clarifying the effects of these factors. The inlet and outlet gas flow is the same on the small flows used, whereby no pressure occurs in the reactor pipe.

The reactor itself is a quartz glass tube reactor, in which the quantity of the catalyst filling is 1-2 ml. The catalyst is heated in an oven, in which the reactor tube is placed inside a steel tube. The ends of the steel tube are joined with aluminium ears to the poles of the current source, i.e. the tube acts per se as a resistance. The oven is controlled by Eurotherm's controller.

After the oven, the device is connected with an on-line principle to HP's gas chromatograph and an integrator.

Samples are taken with a logic controlled repeater motor from the gas flow and the extra gas flow is led to air conditioning.

Air and nitrogen are used for regenerating and flushing the catalyst.

Tables 1A-1C list the test results when using catalysts according to Example 1 on three different metal contents. Table 2 lists the test results when using catalysts according to Example 2. Tables 3 and 4 list the test results when using referance catalysts according to Examples 3 and 4.

In the Tables, the activity is always shown in the units g of converged propylene/g of metal x h. The percentages of the product distributions are percentages by weight.

TABLE 1 A

| Time (h) | Ethene (%) | Propylene (%) | Butenes (%) | Other (%) | Conversion (%) | Activity (g/gh) |
|---|---|---|---|---|---|---|
| 1 | 6.957 | 40.284 | 22.749 | 30.01 | 59.72 | 56.67 |
| 4 | 3.747 | 51.503 | 13.977 | 30.773 | 48.50 | 46.03 |
| 6 | 4.923 | 49.303 | 16.698 | 29.076 | 49.60 | 48.11 |
| 9 | 5.206 | 50.399 | 17.296 | 27.099 | 49.60 | 47.07 |
| 12 | 4.645 | 53.287 | 16.411 | 25.117 | 46.17 | 43.82 |
| 14 | 5.75 | 51.780 | 17.817 | 24.828 | 48.22 | 45.76 |
| 18 | 5.125 | 53.575 | 17.349 | 23.951 | 46.43 | 44.06 |
| 23 | 5.125 | 54.048 | 17.349 | 23.478 | 45.95 | 43.61 |
| 25 | 5.087 | 54.683 | 16.924 | 23.306 | 45.32 | 43.01 |

Catalyst $W(O)(t\text{-}BuCH_2O)_mCl_n$ / silica-alumina (Grace) m = 1-4, n = 4-m

Metal content 3.6%

TABLE 1 B

| Time (h) | Ethene (%) | Propylene (%) | Butenes (%) | Other (%) | Conversion (%) | Activity (g/gh) |
|---|---|---|---|---|---|---|
| 1 | 3.389 | 74.745 | 11.712 | 11.025 | 25.26 | 46.27 |
| 4 | 5.579 | 74.653 | 18.843 | 1.776 | 25.35 | 46.44 |
| 7 | 5.675 | 74.067 | 19.479 | 1.447 | 25.93 | 47.51 |
| 9 | 6.201 | 72.407 | 20.660 | 1.397 | 25.59 | 50.55 |
| 12 | 6.522 | 70.917 | 21.961 | 1.248 | 29.08 | 53.28 |
| 14 | 6.151 | 72.619 | 20.585 | 1.189 | 27.38 | 50.16 |
| 18 | 6.623 | 70.390 | 22.376 | 1.232 | 19.61 | 54.25 |
| 23 | 6.856 | 69.605 | 22.916 | 1.218 | 30.40 | 55.69 |
| 27 | 6.753 | 66.971 | 22.655 | 1.148 | 30.03 | 55.02 |

Metal content 3.6%

Catalyst $W(O)(t\text{-}BuCH_2O)_mCl_n$ / silica + 1.5% of MgO (m = 1-4, n = 4-m)

TABLE 1 C

| Time (h) | Ethene (%) | Propylene (%) | Butenes (%) | Other (%) | Conversion (%) | Activity (g/gh) |
|---|---|---|---|---|---|---|
| 1 | 14.657 | 51.898 | 32.478 | 0.967 | 48.10 | 11.25 |
| 4 | 13.591 | 50.878 | 34.591 | 0.940 | 49.12 | 11.49 |
| 7 | 12.576 | 52.221 | 34.263 | 0.040 | 47.78 | 11.17 |
| 9 | 12.424 | 62.687 | 33.957 | 0.932 | 47.31 | 11.06 |
| 12 | 12.279 | 54.477 | 32.296 | 0.048 | 42.52 | 10.64 |
| 14 | 12.115 | 54.873 | 32.058 | 0.965 | 56.13 | 10.55 |
| 22 | 11.070 | 56.064 | 31.025 | 0.041 | 43.94 | 10.27 |
| 27 | 11.608 | 57.116 | 30.331 | 0.945 | 42.88 | 10.03 |

Metal content 13.5%

Catalyst $W(O)(t\text{-}BuCH_2O)_mCl_n$ / silica, (m = 1-4, n = 4-m)

TABLE 2

| Time (h) | Ethene (%) | Propylene (%) | Butenes (%) | Other (%) | Conversion (%) | Activity (g/gh) |
|---|---|---|---|---|---|---|
| 1 | 10.678 | 53.261 | 34.568 | 1.493 | 46.74 | 26.95 |
| 4 | 10.636 | 53.622 | 34.469 | 1.273 | 46.38 | 26.74 |
| 7 | 10.758 | 53.262 | 34.272 | 1.707 | 46.74 | 26.15 |
| 9 | 10.654 | 64.842 | 13.979 | 1.536 | 46.16 | 26.62 |
| 12 | 10.154 | 56.023 | 32.530 | 1.293 | 43.98 | 25.36 |
| 18 | 9.138 | 60.309 | 29.324 | 1.229 | 39.69 | 22.81 |
| 23.3 | 9.170 | 59.990 | 29.597 | 1.243 | 40.01 | 23.07 |
| 28.3 | 8.046 | 64.877 | 26.216 | 0.861 | 35.12 | 20.25 |

Metal content 6.5%

Catalyst $W(O)(t\text{-}BuCH_2O)_3Br_2$ / silica

TABLE 3 (Comparison)

| Time (h) | Ethene (%) | Propylene (%) | Butenes (%) | Other (%) | Conversion (%) | Activity (g/gh) |
|---|---|---|---|---|---|---|
| 0.5 | 7.390 | 67.688 | 24.033 | 0.949 | 32.31 | 14.76 |
| 4 | 10.759 | 59.471 | 28.846 | 0.924 | 40.53 | 18.52 |
| 7 | 11.015 | 58.608 | 29.466 | 0.511 | 41.39 | 18.91 |
| 9.5 | 10.046 | 58.570 | 29.580 | 0.904 | 41.43 | 18.53 |
| 12 | 11.082 | 58.474 | 29.541 | 0.503 | 41.53 | 18.97 |
| 14 | 10.756 | 59.178 | 29.166 | 0.900 | 40.82 | 18.65 |
| 20 | 9.649 | 62.747 | 26.740 | 0.864 | 37.25 | 25.11 |

Metal content 4.9%

Catalyst $WO_3$ / silica

TABLE 4

| Time (h) | Ethene (%) | Propylene (%) | Butenes (%) | Other (%) | Conversion (%) | Activity (g/gh) |
|---|---|---|---|---|---|---|
| 1 | 5.182 | 80.536 | 13.362 | 0.920 | 19.46 | 7.69 |
| 4 | 9.030 | 66.476 | 23.209 | 1.286 | 33.52 | 13.25 |
| 7 | 10.034 | 62.474 | 25.985 | 1.507 | 37.53 | 14.83 |
| 9 | 10.376 | 65.459 | 26.643 | 1.522 | 38.54 | 15.23 |
| 12 | 10.417 | 50.571 | 27.400 | 1.512 | 39.33 | 15.54 |
| 15 | 10.501 | 60.126 | 27.868 | 1.505 | 35.87 | 15.76 |
| 20 | 12.330 | 54.163 | 30.134 | 3.373 | 45.84 | 18.11 |
| 24 | 12.755 | 53.369 | 30.259 | 3.617 | 46.63 | 18.43 |
| 25 | 12.762 | 53.410 | 30.365 | 3.463 | 46.59 | 18.41 |

Metal content 5.9%

Catalyst $WO_3$ / silica + 1.5% of MgO

## Claims

1. A catalyst system for metathesis reactions of olefines, which catalyst system comprises a metal carbene complex on a solid carrier, in which the metal carbene complex has the formula:
$$M(O)_y(RCH_2O)_mX_{n9}$$
wherein M is W or Mo; R is t-butyl; y is 0 or 1; and when y is o, m = 3 or 4, X is Br and n is from 5 to m; and when y is 1, m is from 1 to 4, X = Cl and n is from 4 to m.

2. A catalyst system according to Claim 1, in which the metal carbene complex has the formula: $W(RCH_2O)_3Br_2$ or $W(RCH_2O)_4Br$, wherein R is t-butyl.

3. A catalyst system according to Claim 1, in which the metal carbene complex has the formula: $W(O)(RCH_2O)_mC1_{n9}$ wherein R is t-butyl, m is from 1 to 4 and n is from 4 to m.

4. A catalyst system acording to any one of the preceding claims in which the carrier comprises one or more of silica, alumina and magnesium oxide.

5. A catalyst system according to Claim 4, in which the carrier is silica or a mixture of silica and magnesium oxide.

6. A catalyst system according to any one of the preceding claims, in which the metal-carbene-complex-containing carrier is calcined by heating in a nitrogen, oxygen or hydrocarbon atmosphere.

7. A catalyst system as claimed in Claim 1 substantially as hereinbefore described with reference to the examples.

8. A process for the metathesis of an olefine in the presence of a catalyst in which the catalyst is as claimed in any one of the preceding claims.

9. A process as claimed in Claim 8 in which the olefine is propylene.

10. A process as claimed in Claim 8 substantially as herebefore described.

European Patent Office

# EUROPEAN SEARCH REPORT

Application Number

EP 91 30 3648

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 283 400 (SOCIETE NATIONALE ELF AQUITAINE) --- | | C 07 C 6/04<br>B 01 J 31/12 |
| A | US-A-4 060 468 (K.F. CASTNER) --- | | |
| A | DE-A-1 022 205 (BAYER) --- | | |
| A | EP-A-0 218 138 (MASSACHUSSETTS INSTITUTE OF TECHNOLOGY) ----- | | |
| | | | TECHNICAL FIELDS SEARCHED (Int. Cl.5) |
| | | | C 07 C<br>B 01 J |

The present search report has been drawn up for all claims

| Place of search | Date of completion of the search | Examiner |
|---|---|---|
| THE HAGUE | 19-08-1991 | THION M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)